(19) Europäisches Patentamt
European Patent Office
Office européen des brevets

(11) Veröffentlichungsnummer: **0 000 907**
**B1**

(12) # EUROPÄISCHE PATENTSCHRIFT

(45) Veröffentlichungstag der Patentschrift: **17.06.81**

(51) Int. Cl.³: **C 07 D 307/32, C 07 D 307/60 //A23L 1/226**

(21) Anmeldenummer: **78100650.7**

(22) Anmeldetag: **11.08.78**

(54) **Verfahren zur Herstellung von Furanonen und Zwischenprodukte in diesem Verfahren.**

(30) Priorität: **11.08.77 LU 77955**
**14.12.77 LU 78691**

(43) Veröffentlichungstag der Anmeldung:
**07.03.79 Patentblatt 79/5**

(45) Bekanntmachung des Hinweises auf die Patenterteilung:
**17.06.81 Patentblatt 81/24**

(84) Benannte Vertragsstaaten:
**BE CH DE FR GB NL**

(56) Entgegenhaltungen:
**DE - A - 1 913 476**
**DE - A - 2 600 864**
**DE - B - 1 768 649**
**DE - B - 1 768 658**
**DE - B - 1 793 444**
**DE - B - 2 105 014**
**NL - A - 72 16271**

**CHEMICAL ABSTRACTS, (1972), 76 152984f "Tautomerism of 4-cyanotiophan-3-one and 4-cyano-5-methyltetrahydrofuran-3-one", S. N. MALKOVA Erastov, O. A. Ignat'eva.**

**HOUBEN-WEYL, 4-Auflage, 1965. "Methoden der organischen Chemie", Georg Thieme Verlag, Stuttgart, Band V1/3, Seiten 587—588**

(73) Patentinhaber: **L. Givaudan & Cie Société Anonyme**
**Patentdienst Postfach**
**CH-4002 Basel (CH)**

(72) Erfinder: **Huber, Ulrich, Dr.**
**Haselweg 3**
**CH-8032 Zürich (CH)**
Erfinder: **Wild, Hans Jakob, Dr.**
**Seehaldenweg 34**
**CH-8706 Meilen (CH)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 907 B1

# Verfahren zur Herstellung von Furanonen und Zwischenprodukte in diesem Verfahren

Die Erfindung betrifft ein neues Verfahren zur Herstellung von Furanonen, und zwar solchen der allgemeinen Formel

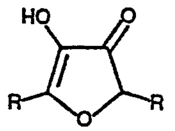

I

worin R Wasserstoff, Methyl oder Aethyl bedeutet; die Reste R können gleich oder verschieden sein.

Das erfindungsgemässe Verfahren ist dadurch gekennzeichnet, dass man eine Verbindung der allgemeinen Formel

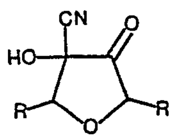

II

worin R obige Bedeutung besitzt, der thermischen oder basenkatalysierten Cyanhydrinspaltung unterwirft.

Im Falle der thermischen Durchführung erhitzt man die Verbindung der Formel II zweckmässigerweise auf Temperaturen von ca. 50 bis 400°C, insbesondere auf ca. 80—250°C.

Im Falle der basenkatalytischen Cyanhydrinspaltung versetzt man die Verbindung der Formel II, zweckmässigerweise mit katalytischen Mengen, z.B. mit 1/1000—1/10 Aequivalenten, oder aber auch mit grösseren, z.B. molaren Mengen einer Base.

Die Natur der Base ist nicht kritisch. Als Basen kommen z.B. in Frage: anorganische Basen wie Alkalimetallhydroxyde, z.B. NaOH, Erdalkalimetallhydroxyde, z.B. $Ca(OH)_2$, $Mg(OH)_2$, Alkalimetallcarbonate, z.B. $Na_2CO_3$, $K_2CO_3$, Alkalimetallbicarbonate wie $NaHCO_3$, Ammoniak, andere basische Salze wie $Na_3PO_4$, $K_2HPO_4$, Borax, basische Puffersysteme wie z.B. $NaHCO_3/Na_2CO_3$, $K_2HPO_4/K_3PO_4$, etc., organische Basen wie Amine, z.B. Triäthylamin, Pyridin, Morpholin, etc., Salze organischer Säuren mit starken Basen, wie Natriumacetat, -format, -oxalat, -citrat, -lactat, oder basische Ionentauscher, z.B. Amberlite JRA 400®, Dowex 2®, etc.

Die Cyanhydrinspaltung kann in der Gasphase oder in flüssiger Phase durchgeführt werden. Anwesenheit eines Lösungsmittels ist nicht erforderlich, jedoch zweckmässig.

Als Reaktionstemperaturen kommen insbesondere 50—200°C, bevorzugt ca. 100°C in Frage.

Die Natur des Lösungsmittels ist nicht kritisch, es können polare Lösungsmittel — wie Wasser, Ammoniak, Alkohole, oder apolare Lösungsmittel, wie Toluol, Benzol, Toluol, Aether, Petroläther, etc., zur Verwendung gelangen.

Bevorzugte Systeme sind basische Ionentauscher in der $OH^{\ominus}$ — Form/$H_2O$, oder Salze organischer Säuren, wie Natriumoxalat/$H_2O$, oder Pyridin/Toluol bei Reaktionstemperaturen von rund 100°C.

Die Verbindungen der Formel II sind neu. Sie bilden ebenfalls Gegenstand der vorliegenden Erfindung.

Die Verbindungen der Formel II können vorteilhaft durch Oxydation von Verbindungen der Formel

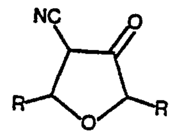

III

worin R obige Bedeutung besitzt, erhalten werden.

Als Oxydationsmittel sind insbesondere Alkalimetallcaroate, z.B. $KHSO_5$, geeignet. Bevorzugt ist "Caroat"® ($KHSO_5$ mit geringen Mengen $KHSO_4$ und $K_2SO_4$).

Das Caroat wird zweckmässigerweise in einer Menge von 1—2,5 Aequivalenten, insbesondere 1,1—1,5 Aequivalenten, verwendet.

Die Oxydation wird vorzugsweise in polaren Lösungsmitteln, wie Wasser, Alkoholen, Aceton, Acetonitril, oder Gemischen solcher Lösungsmittel durchgeführt.

Der pH-Wert des Mediums beträgt zweckmässigerweise ca. 3—11, wie dies durch entsprechende Puffersysteme vom Carbonat-, Phosphat-, Citrat-, Borat-, $NH_3/NH_4^+$- oder Oxalattyp in an sich bekannter Weise erzeugt werden kann.

Die Reaktionstemperatur kann beispielsweise zwischen −10 bis 60°C, bevorzugt zwischen 0 bis 20°C liegen.

Die Verbindungen der Formel III können vorteilhaft durch Umsetzung einer Verbindung der Formel

R—CH=CH—CN IV

mit einer Verbindung der Formel

OH
|
$R—CH—COOR_1$ V

worin R obige Bedeutung besitzt und $R_1$ nieder-Alkyl darstellt,
erhalten werden.

Die Umsetzung der substituierten Acrylsäurenitrils IV mit dem Säureester V erfolgt zweckmässigerweise bei erhöhter Temperatur, beispielsweise bei 40—100°C, insbesondere bei Temperaturen um 60°C. Die Umsetzung lässt sich auch bei tieferer Temperatur, z.B. bei Zimmertemperatur durchführen. Bei dieser Temperatur wird aber die Bildung von Nebenprodukten beobachtet, welche bei der Aufarbeitung von III wieder entfernt werden

müssen, z.B. durch Auswaschen in schwach basischem Milieu.

Das molare Verhältnis von Verbindung IV zu Verbindung V beträgt vorzugsweise 1:1.

Man arbeitet zweckmässigerweise in Gegenwart einer Base und in einem Lösungsmittel.

Als Base werden zweckmässigerweise 1—2 Aequivalente, insbesondere 1—1,3 Aequivalente einer starken Base, z.B. eines Hydrids wie NaH, eines Amids wie $KNH_2$,

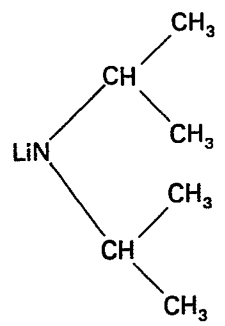

etc. eines Hydroxyds wie NaOH, eines Alkoholats wie $NaOC_2H_5$, KO

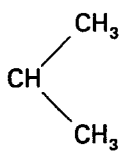

oder eines Metalls wie Na, K verwendet.

Als Lösungsmittel kommen insbesondere polare, bevorzugt aprotische Lösungsmittel in Frage.

Beispiele sind Aether, wie Tetrahydrofuran, Dioxan, Diglyme, Diäthyläther, Diisopropyläther, Nitroverbindungen wie Nitromethan, Nitrobenzol, Nitrile wie Acetonitril, oder Dimethylformamid, Dimethylsulfoxyd, etc. Speziell bevorzugt sind Aether.

Beispiele von protischen Lösungsmitteln sind Alkohole wie tert. Butylalkohol oder Isopropanol.

Die Verbindungen der Formel I sind bekannt; sie stellen Geschmackstoffe dar. Sie wurden bisher beispielsweise durch Ozonisierung von Alkindiolen und säure Cyclisierung der erhaltenen Dioxodiole hergestellt. [Re et al., Helv. Chim. Acta *56,* 1882 (1973)]. Dem Verfahren haften die Nachteile an, dass a) primär unstabile Ozonide entstehen (die explodieren können) und die säure Cyclisierungsstufe unbefriedigende Ausbeuten zeitigt.

Beispiel 1

a) Zu 24 g (1 Mol) Natriumhydrid in 500 ml Tetrahydrofuran werden 130 g (1.1 Mol) Milchsäureäthylester zugetropft. Zu der graubraunen Lösung werden nun bei Rückflusstemperatur 73,7 g (1.1 Mol) Crotonsäurenitril in 60 ml Tetrahydrofuran gegeben und das Gemisch weitere 90 Minuten rückflussiert. Die erkaltete Reaktionslösung wird mit 250 ml 5n HCl versetzt und dreimal mit Aether extrahiert. Die vereinigten Aetherphasen werden dreimal mit Wasser gewaschen, mit Natriumsulfat getrocknet und eingeengt, wobei man 133,6 g (96% Ausbeute) 2,5-Dimethyl-4-cyano-tetrahydrofuran-3-on erhält, Sdp. 109—111°C/18 Torr. Gaschromatographische Identifizierung (GC) an 3m-Säule, 2% Carbowax auf Chromosorb: bei 200°C werden Retentionszeiten von 2.9 und 3.2 Minuten gemessen (Diastereomerengemisch), $n_D^{20} = 1.450$.

b) In einem Kolben werden 7 g des oben erhaltenen cyclischen Nitrils und 21 g $NaHCO_3$ in 400 ml Wasser gelöst und bei 10°C mit einer Lösung von 25 g "Caroat" (Degussa) in 80 ml Wasser versetzt und nach 30 Minuten viermal mit je 150 ml Essigester extrahiert. Die vereinigten organischen Phasen werden über $Na_2SO_4$ getrocknet und eingeengt, wobei 4,3 g eines Diastereomerengemisches von 2,5-Dimethyl - 4 - hydroxy - 4 - cyano - tetrahydrofuran - 3 - on (55% Ausbeute) erhalten werden. Sdp. ~ 95°/0.04 Torr, $n_D^{20} = 1.468$.

*IR* 3400 nm (stark), 3010 nm, 2970 und 2910 nm (Dublett), 2280 nm (schwach), 1790 nm (mittel), 1385 nm (stark), 1110 nm (stark).

$c_1$) 5 g des obigen Cyanhydrins und 7.5 g Ionentauscher Dowex 2 ($OH^{\ominus}$Form) werden während einer Stunde in 30 ml Wasser rückflussiert, darauf wird filtriert. Das Filtrat wird mit Kochsalz gesättigt und viermal mit je 80 ml Essigester extrahiert. Die vereinigten, über $Na_2SO_4$ getrockneten, organischen Phasen werden eingeengt und ergeben 2 g (50% Ausbeute) eines Oeles, welches beim Stehen auskristallisiert. Durch Dünnschichtchromatographie und NMR wird Identität mit 4-Hydroxy-2,5-dimethyl-3(2H)-furanon nachgewiesen.

$c_2$) 1 g des obigen Cyanhydrins und 0,7 g Triäthylamin werden in 10 ml Toluol während 15 Minuten rückflussiert. Nun gibt man 5 g Kieselgel (Merck) zu und filtriert ab. Die eingeengte Lösung ergibt 0.34 g 4-Hydroxy-2,5-dimethyl-3(2H)-furanon.

Beispiel 2

a) Zu 12 g (0.5 Mol) Natriumhydrid in 500 ml Tetrahydrofuran werden 72.6 g (0.55 Mol) Glykolsäurebutylester getropft. Das entstandene grünbraune Reaktionsgemisch wird unter Rückflusstemperatur mit 36.9 g Crotonsäurenitril in 50 ml Tetrahydrofuran versetzt und 90 Minuten bei Rückflusstemperatur gehalten. Das erkaltete Reaktionsgemisch wird mit $NaHCO_3$-Lösung auf pH 9 gestellt und dreimal mit Aether gewaschen. Die wässerige Phase wird mit HCl auf pH 1 angesäuert und mit Aether viermal extrahiert. Die getrockneten und eingeengten Aetherphasen ergeben 29,1 g des 4-Cyano-5-methyl-tetrahydrofuran-3-ons. Sdp 112—116°C/13 Torr, GC (Carbowax, 180°C) ein Peak, IR (Film): 2250 (m)-CN; 1783 (s)

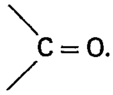

b) In einem Kolben werden 3.1 g des oben erhaltenen cyclischen Nitrils, 10,5 g $NaHCO_3$ und 2 g NaOH in 30 ml Wasser gelöst und bei 15—20°C mit einer Lösung von 11 g Caroat (Degussa) in 35 ml Wasser versetzt. Nach 30 Minuten wird fünfmal mit je 35 ml Essigester extrahiert und die vereinigten Essigphasen werden getrocknet und eingeengt. Es werden 2,2 g (63% der Theorie) 4 - Cyano - 4 - hydroxy - 5 - methyl - tetrahydrofuran-3-on als hellbraunes Oel erhalten; GC: ein Peak. NMR ($CDCl_3$) zeigt komplexe Multipletts zwischen 3.3—4.7 ppm und 1—1.8 ppm sowie ein OH-Signal bei 5.7 ppm.

c) 1.07 g des erhaltenen Cyanhydrins und 2.0 g Natriumglukonat werden in 15 ml Wasser während 15 Minuten rückflussiert. Nun wird fünfmal mit Essigester extrahiert, die Essigesterphasen getrocknet und eingeengt, wobei 0.42 g 4 - Hydroxy - 5 - methyl - 3(2H) - furanon erhalten werden. Das umkristallisierte Material (Smp. 111—120°C) zeigt folgendes NMR ($CDCl_3$): 7.3 ppm (Singulett 1 H, OH): 4.5—4.65 ppm (Multiplett 3 H, $CH_3$).

## Beispiel 3

a) 8,7 g (0,2 Mol) Natriumhydrid werden in 100 ml Tetrahydrofuran suspendiert und bei Raumtemperatur während 90 Minuten mit 23,6 g (0.2 Mol) Milchsäureäthylester gerüht Hierauf werden bei 60°C 9,5 g (0.18 Mol) Acrylnitril zugetropft und das Gemisch wird 90 Minuten rückflussiert. Das Reaktionsgemisch wird auf 200 ml Wasser geworfen, 2 mal mit je 100 ml Aether gewaschen, die wässerige Phase mit 2n Salzsäure auf pH 1 gestellt und 3 mal mit je 150 ml Aether extrahiert. Die getrockneten und eingeengten Aetherphasen ergeben 20,1 g (89%) 4-Cyano - 2 - methyltetrahydrofuran - 3 - on, Sdp. 116—118°/14 mm Hg: IR: 2250 (CN), 1780 (C=O); NMR ($CDCl_3$): 1,37 ppm Dublett ($CH_3$); 3,4—4,9 ppm Multiplett.

b) Das oben erhaltene Produkt wird gemäss Beispiel 2b) behandelt. Man erhält in 71%iger Ausbeute 4 - Cyano - 4 - hydroxy - 2 - methyltetrahydrofuran - 3 - on als Diastereomerengemisch; IR: 3350 (OH); 2290 schwach (CN), 1785 und 1730 (C=N); NMR ($CDCl_3$): 1,4—1,7 ppm Multiplett ($CH_3$); 3,7—4,8 ppm Multiplett (3 × O—C—H), 7,1 Singulett (OH).

c) 3 g der erhaltenen Cyanhydrins werden zusammen mit 2,2 g Natriumacetat in 40 ml Wasser gelöst und während 15 Minuten auf 70°C erhitzt. Darauf wird 5 mal mit je 50 ml Methylenchlorid extrahiert. Die getrockneten und eingeengten Methylenchloridphasen ergeben 150 mg (6%) kristallines 4-Hydroxy-5-methyl-3(2H)-furanon, welches mit jenem in Beispiel 2c) identisch ist.

## Beispiel 4

a) 2,3 g (0,1 Mol) Natrium werden in 50 ml Isopropanol gelöst und unter Kühlung mit 14,5 g (0,11 Mol) 2-Hydroxy-buttersäure-äthylester versetzt. Bei Rückflusstemperatur werden 7,4 g (0,11 Mol) Crotonsäurenitril zugetropft und anschliessend noch 90 Minuten rückflussiert. Das Reaktionsgemisch wird auf 100 ml Wasser gegossen und bei pH 11 2 mal mit je 100 ml Methylenchlorid gewaschen. Die wässerige Phase wird mit konzentrierter Salzsäure auf pH 1 gestellt und 5 mal mit je 100 ml Methylenchlorid extrahiert. Die vereinigten organischen Phasen werden über Natriumsulfat getrocknet und eingeengt und ergeben so 10,2 g (66%) 2-Aethyl-4-cyano-5-methyl-tetrahydrofuran-3-on (Diastereomerengemisch) Sdp. 254°C; IR: 2370 (C=N); 1775 (C=O); MS: 153; 138; 125; 68 (100%).

b) 6 g des erhaltenen Nitrils werden zusammen mit 3,8 g Borax und 3,2 g Natriumhydroxyd in 40 ml Wasser gelöst und portionenweise mit 17 g Caroat versetzt. Nach 30 Minuten wird mit verdünnter Schwefelsäure (1:1) angesäuert und 4 mal mit je 50 ml Essigester extrahiert. Nach Trocknen und Einengen werden 5,5 g (83%) 2 - Aethyl - 4 - cyano - 4-hydroxy - 5 - methyl - tetrahydrofuran - 3 - on erhalten. IR: 3350 (OH), 2240 schwach (CN), 1780 und 1720 (C=O); NMR ($CDCl_3$): 0,8—2,2 ppm Multiplett, 3,5—4,9 ppm Multiplett, 6,2 Singulett (OH).

c) 5 g des erhaltenen Materials werden in 40 ml Wasser gelöst mit 2n Schwefelsäure auf pH 1 gestellt. Nach 4 stündigem Rückflussieren wird abgekühlt und hierauf 5 mal mit je 50 ml Methylenchlorid extrahiert. Die Methylenchloridphasen werden über Natriumsulfat getrocknet und eingeengt und ergeben so 2,6 g (63%) eines Gemisches von 2 - Aethyl-4 - hydroxy - 5 - methyl - 3(2H) - furanon und 5 - Aethyl - 4 - hydroxy - 2 - methyl - 3(2H)-furanon. MS: 142 (100%), 127, 114, 99, 85, 71, 57, 4. IR: 3250 (OH), 1690 (C=O), 1615 stark (C=C).

## Beispiel 5

a) Werden in Beispiel 1a) anstelle des Crotonsäurenitrils 89,1 g (1,1 Mol) 2-Pentensäurenitril (hergestellt nach D. Mac Peek et. al., J. Am. Chem. Soc. *81,* 680 [1959]) verwendet, erhält man in 54%iger Ausbeute 5-Aethyl - 4 - cyano - 2 - methyl - tetrahydrofuran-3-on. $n_D^{20}$ = 1,4552; IR: 2250 (CN), 1778 (C=O); MS: 153, 125, 96, 82 (100%).

b) Das erhaltene Nitril wird gemäss Beispiel 1b) mit Caroat behandelt und ergibt in 83%iger Ausbeute zähflüssiges 5 - Aethyl - 4 - cyano-4 - hydroxy - 2 - methyltetrahydrofuran - 3 - on. $n_D^{20}$ = 1,4587; IR: 3400 (OH), 2240 (schwach, CN), 1785 (C=O); MS: 142 (M—HCN), 114, 97, 82, 70 (100%).

c) Das Cyanhydrin wird gemäss Beispiel 1c) behandelt. Man erhält in 84%iger Ausbeute 5-Aethyl - 4 - hydroxy - 2 - methyl - 3(2H)-

furanon. $n_D^{20}$ = 1,5071. Die Verbindung isomerisiert beim Stehenlassen teilweise (siehe Beispiel 4c)).

Beispiel 6

a) Wird in Beispiel 3a) der Milchsäureäthylester durch $\alpha$ - Hydroxy - buttersäureäthylester und das Acrylonitril durch 2-Pentensäurenitril (hergestellt nach D. Mac Peek et. al., J. Amer. Chem. Soc. *81*, 680 [1959]) ersetzt, so erhält man neben wenig dimerem Pentensäurenitril [welches sich aus der basischen Phase abtrennen lässt] in 50%iger Ausbeute 2,5 - Diäthyl - 4 - cyanotetrahydrofuran - 3 - on. $n_D^{20}$ = 1,4538; IR: 2250 (CN); 1775 (C=O). NMR ($CDCl_3$): 3,5—4,6 (M/2 Pr (H2, H5); 3,25 D und 3,08 D/1 Pr (H4); 1,4—2,1 M/4 Pr (2 × $CH_2$); 0,8—1,3 M/6 Pr (2 × $CH_3$); MS: 167 ($M^+$), 139, 82 (100%).

b) Das erhaltene Nitril wird analog Beispiel 2b) oxydiert. Man erhält in 79%iger Ausbeute zähflüssiges 2,5-Diäthyl - 4 - cyano - 4-hydroxytetrahydrofuran - 3 - on. $n_D^{20}$ = 1,4588; IR: 3300 (OH) 2240 (schwach, CN), 1782 (C=O); MS: 156 (M—HCN), 97, 82, 70 (100%).

c) Das erhaltene Cyanhydrin wird analog Beispiel 3c) gespalten. Man erhält in 75%iger Ausbeute, 2,5-Diäthyl - 4 - hydroxy - 3(2H) - furanon. Sdp. 50—55°C/0,03 mm Hg. Nach Umkristallisation aus Diisopropyläther beträgt der Schmelzpunkt 94—96°C; IR: 3250 (OH), 1690 (C=O), 1620 (C=C); NMR ($CDCl_3$): 7,3 ppm S/1 Pr (OH), 4,37 Tr breit/1 Pr (H2), 2,65 Quart/2 Pr ($CH_2$ an C5), 1,5—2,2 M/2 Pr ($CH_2$ an C2), 0,8—1,4 2 × Tr/6 Pr (2 × $CH_3$); MS: 156 ($M^+$), 141, 99, 71, 58 (100%).

Beispiel 7

a) Wird in Beispiel 3a) Milchsäureäthylester durch $\alpha$-Hydroxybuttersäureäthylester ersetzt, so erhält man in 68%iger Ausbeute 2 - Aethyl - 4 - cyanotetrahydrofuran - 3 - on. $n_D^{20}$ = 1,4641; IR: 2260 (CN), 1775 (C=O); MS: 139 ($M^+$), 111 (M—CO), 107, 57, 54 (100%).

b) Das Nitril wird analog Beispiel 2b) oxydiert. Man erhält in 50%iger Ausbeute 2-Aethyl-4-cyano - 4 - hydroxytetrahydrofuran - 3 - on gelbes Oel. $n_D^{20}$ = 1,4586, IR: 3400 (OH), 2250 (schwach, CN), 1783 (C=O); NMR ($CDCl_3$): 4,70 ppm D/1 Pr (H5), 3,9—4,4 M/2 Pr (H2 und OH), 3,88 D/1 Pr (H5), 1,5—2,2 M/2 Pr ($CH_2$ an C2), 1,05 Tr/3 Pr ($CH_3$).

c) Das erhaltene Cyanhydrin wird analog Beispiel 2c) behandelt. Man erhält in 41%iger Ausbeute 5 - Aethyl - 4 - hydroxy - 3(2H) - furanon. Smp. 47—48°C (aus Diisopropyläther); IR ($CHCl_3$): 3250 (OH), 1710 (C=O), 1620 (C=C); NMR ($CDCl_3$): 6,4 ppm S breit/1 Pr (OH), 4,53 S/2 Pr (H2), 2,69 Quart/2 Pr ($CH_2$ an C5), 1,27 Tr/3 Pr ($CH_3$).

**Patentansprüche**

1. Verfahren zur Herstellung von Verbindungen der allgemeinen Formel

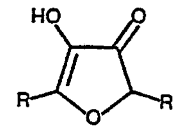

I

worin die Reste R gleich oder verschieden sind und Wasserstoff, Methyl oder Aethyl bedeuten, dadurch gekennzeichnet, dass man eine Verbindung der allgemeinen Formel

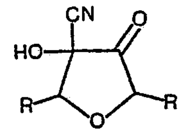

II

worin R obige Bedeutung besitzt, der thermischen oder basenkatalysierten Cyanhydrinspaltung unterwirft.

2. Verfahren nach Anspruch 1, dadurch gekennzeichnet, dass man die Verbindung der Formel II durch Oxydation einer Verbindung der allgemeinen Formel

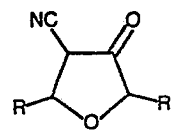

III

worin R die in Anspruch 1 angegebene Bedeutung besitzt, gewinnt.

3. Verfahren nach Anspruch 2, dadurch gekennzeichnet, dass man ein Alkalimetall-Caroat als Oxydationsmittel verwendet.

4. Verfahren nach Anspruch 2 oder 3, dadurch gekennzeichnet, dass man die Verbindung der Formel III durch Umsetzung einer Verbindung der Formel

R—CH=CH—CN IV

mit einer Verbindung der Formel

$$R—CH(OH)—COOR_1 \quad V$$

worin R die in Anspruch 1 angegebene Bedeutung besitzt und $R_1$ nieder-Alkyl darstellt, gewinnt.

5. Verfahren nach Anspruch 4, dadurch gekennzeichnet, dass man die Umsetzung bei erhöhter Temperatur durchführt.

6. Verfahren nach einem der Ansprüche 1 bis 5, dadurch gekennzeichnet, dass R Methyl darstellt.

7. Verbindungen der allgemeinen Formel

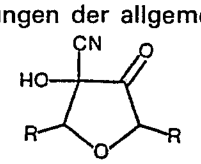

II

worin die Reste R gleich oder verschieden sind und Wasserstoff, Methyl oder Aethyl darstellten.

8. 2,5 - Dimethyl - 4 - cyano - 4 - hydroxytetrahydrofuran - 3 - on.

9. 4 - Cyano - 4 - hydroxy - 5 - methyltetrahydrofuran - 3 - on.

10. 2 - Aethyl - 4 - cyano - hydroxy - 5-methyl - tetrahydrofuran - 3 - on.

11. 4 - Cyano - 4 - hydroxy - 2 - methyl-tetrahydrofuran - 3 - on.

12. 5 - Aethyl - 4 - cyano - 4 - hydroxy-2 - methyl - tetrahydrofuran - 3 - on.

13. 2,5 - Diäthyl - 4 - cyano - 4 - hydroxytetrahydrofuran - 3 - on.

14. 2 - Aethyl - 4 - cyano - 4 - hydroxytetrahydrofuran - 3 - on.

**Revendications**

1. Procédé pour la préparation de composés de formule générale

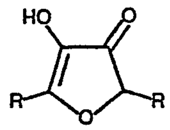

I

dans laquelle chacun des radicaux R, identiques ou différents, représente de l'hydrogène ou un groupe méthyle ou éthyle, caractérisé par le fait que l'on provoque, dans un composé de formule générale

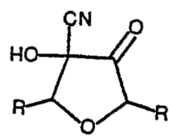

II

dans laquelle R a la signification précitée, l'élimination thermique ou catalysée par une base du groupe cyanhydrine.

2. Procédé suivant la revendication 1, caractérisé par le fait qu'on obtient le composé de formule II par oxydation d'un composé de formule générale.

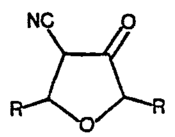

III

dans laquelle R a la signification indiquée à la revendication 1.

3. Procédé suivant la revendication 2, caractérisé par le fait qu'on utilise comme oxydant un caroate de métal alcalin.

4. Procédé suivant la revendication 2 ou 3, caractérisé par le fait qu'on obtient le composé de formule III par réaction d'un composé de formule

R—CH=CH—CN (IV)

avec un composé de formule

$$R\text{—}\underset{|}{\overset{OH}{CH}}\text{—}COOR_1 \qquad (V)$$

formules dans lesquelles R a la signification indiquée à la revendication 1 et $R_1$ représente un groupe alkyle inférieur.

5. Procédé suivant la revendication 4, caractérisé par la fait qu'on effectue la réaction à température élevée.

6. Procédé suivant l'une des revendications 1 à 5, caractérisé par le fait que, dans toutes les formules, R représente le groupe méthyle.

7. Composés de formule générale

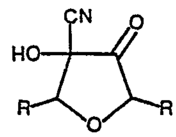

II

dans laquelle chacun des radicaux R, identiques ou différents, représente de l'hydrogène ou un groupe méthyle ou éthyle.

8. La 2,5 - diméthyl - 4 - cyano - 4-hydroxy - tétrahydrofuran - 3 - one.

9. La 4 - cyano - 4 - hydroxy - 5 - méthyl-tétrahydrofuran - 3 - one.

10. La 2 - éthyl - 4 - cyano - 4 - hydroxy - 5-méthyl - tétrahydrofuran - 3 - one.

11. La 4 - cyano - 4 - hydroxy - 2 - méthyl-tétrahydrofuran - 3 - one.

12. La 5 -éthyl - 4 - cyano - 4 - hydroxy - 2-méthyl - tétrahydrofuran - 3 - one.

13. La 2,5 - diéthyl - 4 - cyano - 4 - hydroxy-tétrahydrofuran - 3 - one.

14. La 2 - éthyl - 4 - cyano - 4 - hydroxy-tétrahydrofuran - 3 - one.

**Claims**

1. A process for the manufacture of compounds of the general formula

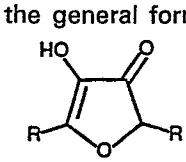

I

wherein the radicals R are identical or different and represent a hydrogen atom or the methyl or ethyl group, which process comprises subjecting a compound of the general formula

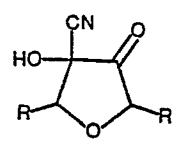

II

wherein R has the above significance, to a thermical or base catalysed cyanohydrin cleavage.

2. A process according to claim 1, wherein a compound of formula II is prepared by oxidising a compound of the general formula

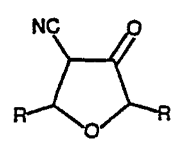

III

wherein R has the significance given in claim 1.

3. A process according to claim 2, wherein an alkali metal caroate is used as the oxidising agent.

4. A process according to claim 2 or claim 3,

wherein a compound of formula III is obtained by reacting a compound of the general formula

$$R—CH{=}CH—CN \qquad (IV)$$

with a compound of the general formula

$$\begin{matrix} & OH & \\ & | & \\ R— & CH & —COOR_1 \end{matrix} \qquad (IV)$$

wherein R has the significance given in claim 1 and $R_1$ represents a lower alkyl group.

5. A process according to claim 4, wherein the reaction is carried out at an elevated temperature.

6. A process according to any one of claims 1 to 5 inclusive, wherein R represents the methyl group.

7. Compounds of the general formula

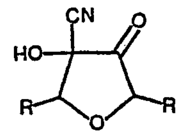

II

wherein the radicals R are identical or different and represent a hydrogen atom or the methyl or ethyl group.

8. 2,5 - Dimethyl - 4 - cyano - 4 - hydroxytetrahydrofuran - 3 - one.

9. 4 - Cyano - 4 - hydroxy - 5 - methyl-tetrahydrofuran - 3 - one.

10. 2 - Ethyl - 4 - cyano - 4 - hydroxy - 5-methyl - tetrahydrofuran - 3 - one.

11. 4 - Cyano - 4 - hydroxy - 2 - methyl-tetrahydrofuran - 3 - one.

12. 5 - Ethyl - 4 - cyano - 4 - hydroxy - 2-methyl - tetrahydrofuran - 3 - one.

13. 2,5 - Diethyl - 4 - cyano - 4 - hydroxytetrahydrofuran - 3 - one.

14. 2 - Ethyl - 4 - cyano - 4 - hydroxy-tetrahydrofuran - 3 - one.